# EUROPEAN PATENT APPLICATION

(11) **EP 2 223 711 A1**
(43) Date of publication of application: **01.09.2010**
(21) Application number: 09152293.8
(22) Date of filing: 06.02.2009
(51) Int. Cl.: A61M 1/00

(54) **Mechanical vacuum source for negative pressure wound therapy and general exudate management**

(71) Applicant: Jap:Ventures Ltd., 92369 Sengenthal (DE)
(72) Inventor: Ahorner-Weber, Johannes Dr., 91052 Erlangen (DE); Wolf, Manfred, 92369 Senenthal (DE); Wiesneth, Juliane, 92369 Sengenthal (DE)
(74) Representative: Vossius & Partner

(57) **Abstract**

The present invention relates to a fluid evacuator for negative pressure wound therapy. The fluid evacuator comprises two main components, namely a container unit (A) and a functional unit (B). The container unit (A) comprises at least a container (14) for the collection of wound fluids. The functional unit (B) is adapted for controlling the pressure inside the container (14), wherein the functional unit (B) comprises a manual pump for creating a vacuum inside the container (14). Moreover, the functional unit (B) further comprising an electric pressure gauge for indicating the pressure inside the container (14).

## Description

The present invention generally relates to wound drainage systems. More particularly, the present invention relates to a vacuum source for negative pressure wound therapy, such as a portable closed-type fluid evacuator for medical use to evacuate drainage fluid (body fluid) from a wound.

### BACKGROUND OF THE INVENTION

For care of surface area injuries with complex tissue defects it is known that the defect is covered over its surface with a film and closed off in an airtight manner. A drainage tube is introduced under the film and connected via an exudate collection container to a vacuum system. Thereby and under pressurization a vacuum is produced in the wound area under the film, which beneficially influences the tissue regeneration and prevents the growth of bacteria. In larger defects it is preferred that a porous foam insert be placed into the wound, into which the drainage tube is inserted, whereby the wound exudate can be siphoned or sectioned off over the overall wound surface area in a more even manner.

According to a well-known method, the vacuum is achieved by an evacuated suction flask, which is evacuated to a high level of vacuum. A flexible tube is connected to the flask and the other end of the tube is introduced under the film. When the line (suction hose) from the flask is opened, the negative pressure prevailing in the flask acts in the wound cavity thus drawing off wound exudate down the line into the flask.

However, it has been shown that such high level vacuums (ca. -600 mmHg/ca. - 0,80 bar) are too high for negative pressure wound therapy applications. Moreover, it has been shown that adjustable and regulateable vacuums, provided by an adjustable vacuum source with a vacuum pump, provide a plurality of advantages over simple evacuated suction flasks.

It is well known in the art to use wound drainage systems with electrical pumping and electronic pressure control systems. The use of such electrical/electronical wound drainage pumps provides the advantage that the pressure level can be controlled and monitored exactly, preferably automatically. However, such electrical/electronical systems are expensive, such that they are typically used in hospitals.

It is also prior art that some all-electrical/electronical pumps are equipped with rechargeable batteries in order to allow the necessary mobilization of the patients. It is unavoidable that the rechargeable batteries have to be connected to a socket for recharging after a few hours of operation.

In case the patient was discharged from hospital to recover at home or at a place where mains are not constantly provided, for example but not only in third world countries, it is desirable to use a cheap and preferably mains-independent vacuum source.

For instance, the vacuum system CombiVac^{®} provides a wound exudate container with integrated manual pump to adjust the vacuum level in the container. The vacuum level, however, is merely very roughly indicated by means of a spring bellows.

EP 186 783 also relates to a fluid evacuator for medical use with a mechanical pump. WO 96/35401 also relates to a similar vacuum system with a manual pump. In contrast to the CombiVac^{®} device, a mechanical pressure gauge is provided. However, such mechanical or aneroid pressure gauges are typically imprecise or large or expensive. In particular, typical bourdon gauges (sometimes called bourdon tube gauges) are inexpensive but typically very large. On the other hand, capsular spring manometers provide a higher accuracy but are typically very expensive. Moreover, the fully mechanical system of WO 96/35401 does not provide any alarm indicating means, which may warn the patient in case of an unexpected pressure drop.

It is therefore an object of the present invention to provide an improved wound drainage vacuum system. In particular, it is an object to provide a preferably cheap wound fluid evacuator with an adjustable and preferably controllable vacuum level. It is yet a further object of the invention to provide a fluid evacuator with a manually and/or automatically, preferably mains-independent, adjustable vacuum level. It is still a further object of the present invention to provide a portable fluid evacuator which reduces a pressure drop and/or eliminates a rapid drop in suction force which might be caused as a result of an increase in quantity of drainage fluid being evacuated from a wound.

These and other objects are achieved by the features of the independent claims. Further preferred embodiments are characterized in the dependent claims.

### SUMMARY OF THE INVENTION

The present invention is directed to a vacuum source for negative pressure wound therapy, such as a wound fluid aspirating device or a fluid evacuator. The device of the present invention comprises preferably two main components, namely a container unit for collecting the wound exudate and a functional unit, preferably with a manual pump for generating the vacuum in the container unit. The functional unit may be integrally formed with the container unit. Alternatively, the two units may be permanently or detachably coupled together. It is further preferred that the two units are airtight connected such that a vacuum may be created inside the container unit. For instance, an integrated system may be constructed as a single use article, such that the contaminated pump and container may be collectively disposed of after use. According to another preferred embodiment of the invention, the functional unit may be constructed for multiple uses and only the container unit is constructed as a disposable article. Alternatively, both the functional unit and the container unit are constructed for multiple uses. The use of a manual pump provides the advantage that the practitioner, or the patient herself/himself, is independent of any stationary vacuum systems of the hospital, so that an ambulatory care of the acutely and/or the chronically ill person is possible. By using a manual pump, the under-pressurization of the container can at any time be created or maintained at a desired level.

The terms "vacuum", "negative pressure" and "under-pressurization" as used in the present invention preferably refer to any gaseous pressure less than the atmospheric pressure. In other words, the term vacuum refers to a negative pressure, in particular to a situation in which an enclosed area has lower pressure than the area around it.

According to a first aspect, the present invention relates to a fluid evacuator for negative pressure wound therapy, which comprises two main components, namely a container unit and a functional unit.

According to a second aspect, the present invention relates to the container unit. The container unit according to the first and second aspects comprises at least a container for the collection of wound exudate.

According to a third aspect, the present invention relates to the functional unit. The functional unit according to the first and third aspects is adapted for controlling the pressure inside the container (unit), wherein the functional unit comprises a manual pump for creating a vacuum inside the container (unit). The functional unit preferably comprises an electronic pressure gauge (also called electronic manometer or electronic vacuum gauge) for indicating the pressure inside the container (unit).

The manual pump provides the advantage that a vacuum can be created even in cases where electricity, like mains or energy from batteries for electrical pumps, is not available. On the other hand, in order to monitor the vacuum level in the container or to adjust the vacuum level to a desired value, the functional unit is equipped with a pressure gauge, preferably an electronic pressure gauge. The electronic pressure gauge (in the following also labelled as electronic vacuum gauge or electronic manometer) provides the further advantage that the practitioner or the patient herself/himself can monitor the current under-pressurization and can, if need be, correct the pressure to the desired value. Since the required electrical power for operating an electrical pressure gauge is lower than the power for operating an electrical pump, it may be sufficient to provide the required electrical power for the pressure gauge by a battery or a plurality of batteries and/or a capacitor. The functional unit may therefore comprise a battery and/or a rechargeable battery and/or a solar cell and/or a capacitor and/or a dynamo (electric generator) for providing the electrical power for the electrical pressure gauge.

The functional unit preferably comprises a housing, wherein the manual pump is operable by means of a lever or crank. Said lever or crank is preferably rotatably or pivotally supported and preferably concealable within said housing. The housing comprises preferably a smooth surface for easy cleaning.

The dynamo is preferably operated by means of a lever or a crank, wherein the lever or crank may be permanently or detachably mounted to the functional unit. According to a preferred embodiment, the lever is mounted at the functional unit in such a way that it disappears within the housing of the functional unit when located in the "unused position". The electrical power generated by means of the dynamo is preferably used to operate electrical pressure gauge and/or to charge a rechargeable battery and/or a capacitor.

The lever or crank for operating the dynamo may be coupled or decoupled with the operation of the mechanical pump mechanism. For instance, in case the vacuum level in the container unit has to be created or adjusted and the battery is still charged sufficiently, it is preferred that the mechanical pump is operated by the lever or crank, but not the dynamo. In case the vacuum level in the container unit has to be created or adjusted and the battery should be charged, it is preferred that the mechanical pump and the dynamo are operated by the lever or crank. In case the vacuum level is correct, but the battery should be charged, it is preferred that the dynamo is operateable by the lever or crank, but not the mechanical pump. According to a preferred embodiment, the above functional unit is constructed such that it may be changed among the above three situations. For instance, the functional unit and/or the container unit may comprise a mechanism which ensures that the container is not evacuated when the lever is operated.

The manual pump comprises preferably a suction means, e.g., a suction bellows, suction bladder, a piston and/or a paddle wheel for evacuating the container. The suction means may be biased, e.g., by means of a spring, to a position with small volume (collapsed/compressed state). In order to create the desired vacuum in the container, the operation of the lever (against the biasing force) enlarges the volume of the suction means (e.g., the suction bellows) such that the suction means is altered into the expanded state. For instance, it is preferred that the lever is biased to extend beyond the housing such that the suction bellows is preferably compressed when the lever extends beyond the housing. The suction bellows preferably expands when the lever moves toward the housing, such that gas, in particular air, is evacuated from the container (unit).

The electrical pressure gauge preferably comprises a digital display for displaying the pressure inside the container. Moreover, the functional unit may additionally or alternatively comprise an electrical and/or mechanical alarm indicator (acoustic and/or visual). It is preferred that the accuracy is at least ±2% F.S. (full scale), more preferably at least ±1% F.S., even more preferably ±0.5% F.S. or better or ±0.2% F.S. or better. It is further preferred that the accuracy class according to DIN1319 or EN 60051 is 2.5 or even better.

Since wound exudate may comprise germs (microorganisms like bacteria) it is preferred that the container unit is germ-proof. In order to allow an evacuation without contamination of the environment, it is further preferred that the container (unit) and/or the functional unit comprises a filter, preferably a hydrophobic germ filter for filtering the air which is evacuated from the container. Said filter may be located within a particular filtration chamber, wherein the filtration chamber is preferably scalable against the container by means of a (mechanically operable) valve. The germ filter assures that contaminated air inside will be filtered before exhaust from the container when the vacuum is generated inside the container. The filter, however, only works sufficiently when it does not get into contact with the wound exudate.

According to a further preferred embodiment, a sachet with a gelling agent is provided inside the container. The sachet is preferably fixedly attached in the container, preferably remote from the inlet and outlet openings of the container unit to avoid a blocking of the inlet or outlet opening by wound exudate or solidified wound exudate. According to a preferred embodiment, the sachet is placed at the bottom of the container.

In order to abruptly let air into the container in case of emergency (e.g., appearance of blood instead of wound exudate) to abort or reduce the suction or negative pressure, a valve, preferably made of plastics with a signal colour, is provided at the functional unit or the container unit. Such an "emergency valve" or "bleed valve'" may also be operated in order to reduce the suction in the container when it was inadvertently pumped beyond the aimed vacuum level.

The container unit may be already equipped with a drainage tube (suction hose), which may be fixedly attached to the container unit.

The container is preferably rigid and preferably made of plastic material, which may be transparent or frosted transparent and preferably comprises a scale for measuring the amount of wound exudate within the container. The container unit and/or the functional unit may be ergonomically formed, preferably kidney-shaped which enables easy carrying.

According to a fourth aspect the fluid evacuator according to the present invention reduces a pressure drop and/or eliminates a rapid drop in suction force which might be caused as a result of an increase in quantity of drainage fluid being evacuated from a wound. In particular, according to a further preferred embodiment, an automatic vacuum adjustment or compensation means is provided inside the container, e.g., at the lower side of the top cover of the container unit. With the automatic vacuum adjustment or compensation means a substantially constant suction force at any time during evacuation of wound fluids may be provided. The gist of the automatic vacuum adjustment or compensation means is to change automatically the active volume inside the container in response to a vacuum level change. For instance, the automatic vacuum adjustment or compensation means may change the active volume by means of a biased surface of the container unit, e.g., a biased lower surface of the cover, wherein the biased (or prestressed) surface regulates the volume inside the container unit in response to the vacuum level inside the container unit. According to a further preferred embodiment, the automatic vacuum adjustment or compensation means may comprise a displacement volume, wherein said displacement volume automatically changes its volume when the pressure inside the container unit increases/decreases.

The automatic vacuum adjustment or compensation means may be constructed in form of a resilient inflatable balloon member. The balloon member may be closed or may comprise at least an opening to the atmosphere such that the interior of the balloon member is in gas communication with the atmosphere. When the gas inside the container unit is discharged to the atmosphere so that the interior of the container is provided with a negative pressure, the balloon member located inside the container is inflated, thereby allowing a constant negative pressure to be maintained at all times by means of the resiliency of the balloon member.

According to another preferred embodiment, the automatic vacuum adjustment or compensation means is constructed as foam or foam member, preferably closed cell foam, e.g. cellular elastomer rubber or caoutchouc/rubber which does not have interconnected pores. The closed cells may be filled with air or with a specialized gas. Again, when the gas inside the container unit is discharged to the atmosphere so that the interior of the container is provided with a negative pressure, the foam with its closed pores located inside the container is inflated due to the relative overpressure in the closed pores. Thereby a substantially constant negative pressure may be maintained at all times by means of the foam member.

The automatic pressure-regulating function by means of the automatic vacuum adjustment or compensation means may be explained as follows. When the vacuum level inside the container increases, the balloon or cellular foam inflates within the container. When drainage fluid is evacuated from the wound through the suction hose by virtue of the negative pressure produced within the container unit, the vacuum level, however, decreases. The negative pressure within the container unit may be maintained at a substantially constant level corresponding to the resiliency of the balloon or foam even when the quantity of received body fluid increases. Unlike conventional fluid evacuators such as simple evacuated bottles (e.g., Redon's bottle), the fluid evacuator according to the present invention reduces the risk of the negative pressure linearly lowering in accordance with the increase in the quantity of body fluid accumulated. Accordingly, the present invention offers great advantages.

According to a fifth aspect, the present invention relates to a method for generating negative pressure, which may be used for negative pressure wound therapy (NPWT) and exudate management; the method comprising the steps: providing a fluid evacuator according to the present invention and operating the manual pump until the electric pressure gauge indicates the desired negative pressure level inside the container unit.

The invention also relates to methods of removing wound secretion/exudate from a wound of a patient. In particular, the present invention also relates to a method for negative pressure wound therapy (NPWT) and exudate management by applying suction to a wound by means of a fluid evacuator as discussed above.

The fluid evacuator according to the present invention has at least the following advantages over the prior art device. The fluid evacuator according to the present invention is preferably completely independent of the power network (mains). Hence, the combination of the container and the functional unit (pump) makes the patient independent of the stationary vacuum system of the hospital, so that an ambulatory care of acute and chronic wounds is possible. By means of the pump, the under-pressurization of the container can at any time be maintained at a desired level; the nurse or the patient can herself/himself monitor the current under-pressurization by means of the preferably precise electronic manometer and can as needed also correct the vacuum to the prescribed level.

With the fluid evacuator according to the present invention, the production of electrical power and vacuum may run concurrently but also independently from each other. Moreover, even a breakdown of an electrical component does not lead to the breakup of the therapy, i.e., it is still possible to produce and to sustain a vacuum if electrical components fail to work. According to a preferred embodiment, the electronic pressure gauge (manometer) and/or the alarm indicating means is/are operated by electrical power, wherein the electrical power may be provided by means of (rechargeable) batteries, capacitors, solar cells or a dynamo. According to a further preferred embodiment, the rechargeable battery of the fluid evacuator may be charged with an external and/or integrated charging device, e.g., a charging device which may be connected to the power network (mains).

Moreover, the fluid evacuator according to the present invention provides an automatic protection of the hydrophobic bacteria filter. Thus, it is possible to carry out the therapy in a safe way and without restrictions or pauses even when the vacuum source is strongly moved, tilted, turned over or lying in the patient's bed someway.

### BRIEF DESCRIPTION OF THE DRAWINGS

The invention will now be described in detail with respect to preferred embodiments with reference to accompanying drawings, wherein:
- Fig. 1: shows an exploded view of a fluid evacuator for negative pressure wound therapy according to the present invention;

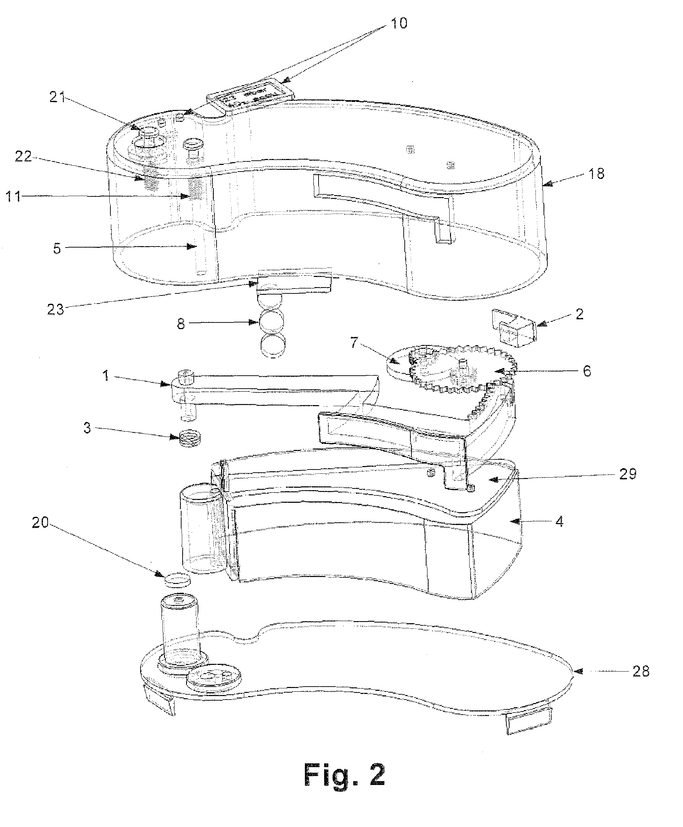

- Fig. 2: shows an exploded view of a functional unit according to the present invention;
- Fig. 3: shows in a perspective elevation details of a functional unit according to the present invention;
- Fig. 4: shows a cross sectional view of a part of the funtional unit with a sealable filtration chamber and pin mechanism; and
- Fig. 5: shows an exploded view of a container unit with a container and a cover, wherein at the lower side of the cover an automatic vacuum adjustment or compensation means is shown.

### DETAILED DESCRIPTION OF THE INVENTION

In the following, the device of the present invention and the method for operating the device will be described.

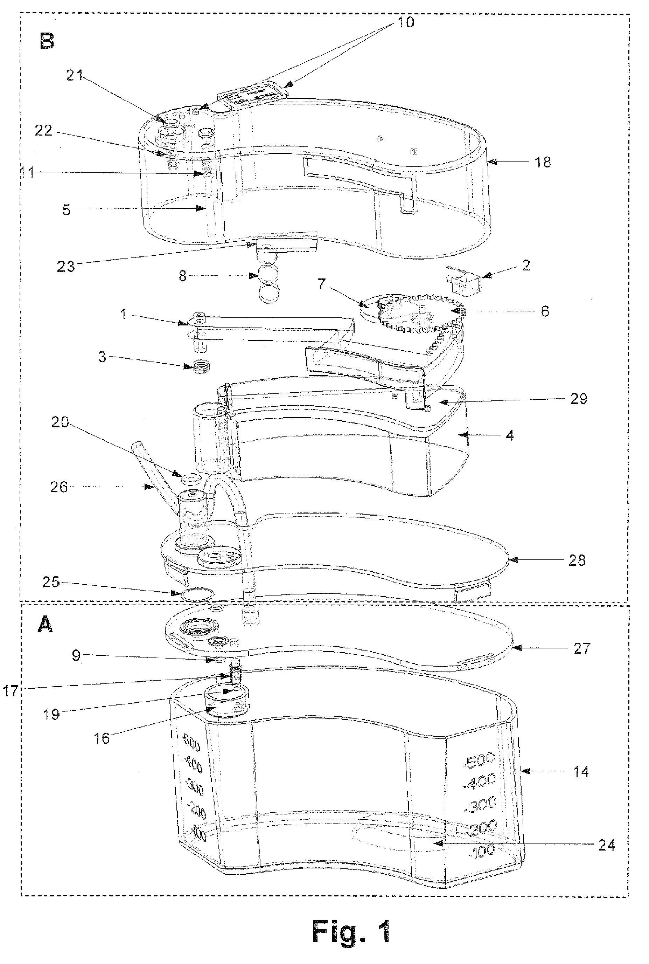

Figure 1 shows an exploded view of a fluid evacuator for negative pressure wound therapy according to a preferred embodiment of the present invention. The fluid evacuator comprises two main components, i.e., a container unit A with a container for collecting the wound fluid (wound exudate) and a functional unit B for controlling the pressure in the container, i.e., for creating and monitoring the vacuum in the container.

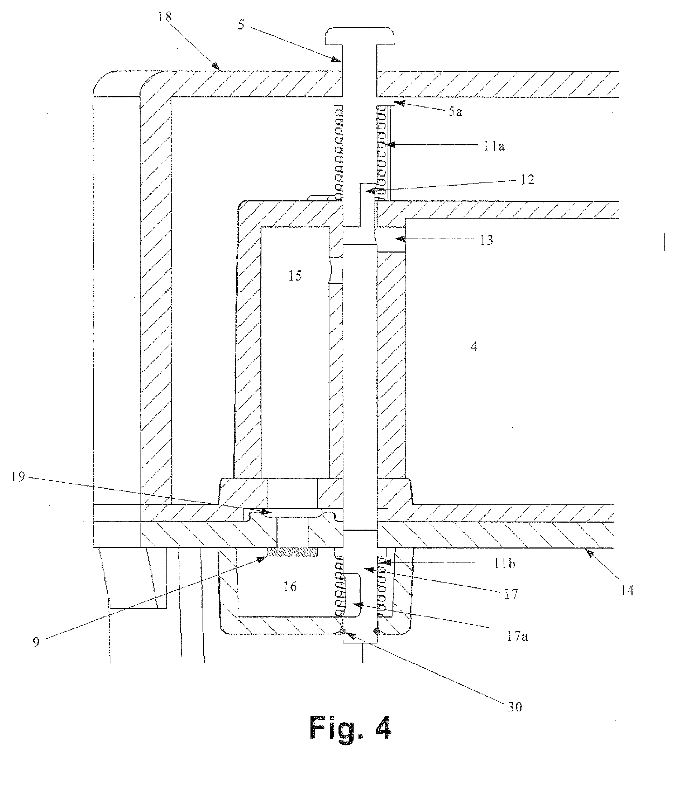

The container unit A comprises a container or receptacle for fluid collection 14 which is covered by a cover 27. The container unit is preferably germ proof when detached from or attached to the functional unit B. An opening at the cover, which may be used for the measurement of the under-pressurization (pressure sensor) is preferably closed by a membrane 25. Other openings may be closable by means of membranes, valves or valve mechanisms (gas communication with the functional unit, e.g., evacuation or emergency air-inlet). For instance, an opening may be provided for a suction hose or drainage tube 26, which consists preferably of plastics and serves the purpose of collecting wound fluid, The suction hose may be fixedly or detachably mounted to the container unit. Moreover, according to a further preferred embodiment, a check valve (one-way valve) may be provided such that wound exudate may enter the container unit but a fatal backflow towards the wound via the suction hose 26 is prevented. The container unit A preferably comprises an opening in order to draw or evacuate the gas from the container. Said opening is preferably equipped with a (hydrophobic bacteria) filter 9. In order to protect the filter 9 from contamination by wound exudate, it is preferred to cover the filter by a filtration chamber, wherein a fluid (gas and/or liquid) communication between the filtration chamber an the inner volume of the container may be operated manually or automatically by a valve (see e.g. Fig. 4).

The container 14 is preferably fully or partly transparent in order to check the wound fluid or the filling level. The container may comprise a scale for reading the filling level, e.g. in millilitre (ml). For instance, the scale may be attached, printed or engraved on the container or produced together with the container, e.g., by injection moulding. The container may have every appropriate form, but is preferably ergonomically kidney-shaped. The volume of the container can be between 50 ml and 1000 ml depending on the mode of application. According to a preferred embodiment, the container is sterilely packed and designed for single use.

A gelling agent 24 with gelling or solidifying or clotting means is preferably strongly affixed, preferably flatly lying on the bottom of the container 14. It is further preferred that the gelling agent is arranged opposite to the inlet opening of the container 14. This fixed arrangement of the gelling agent opposite to the inlet opening, which serves the purpose of application security, ensures that the wound fluid solidifies in each location of the container but beginning from the bottom so that the fluid access into the container is not blocked.

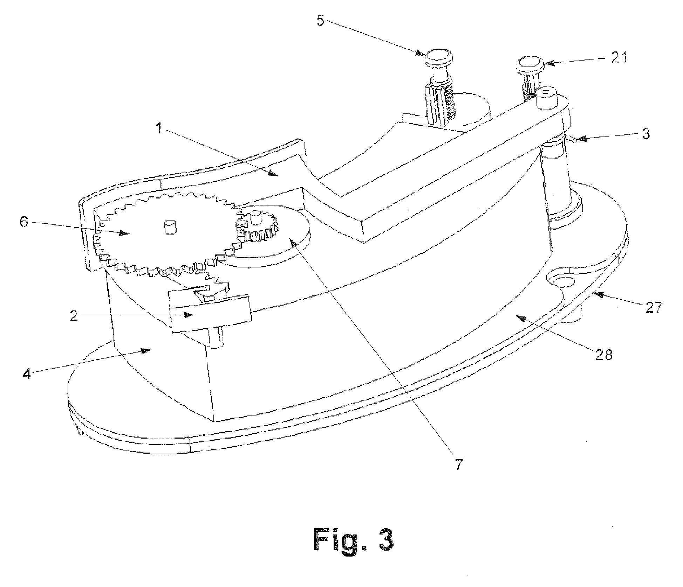

On the functional unit B there is a means for mechanically producing the vacuum. The functional unit B comprises at the bottom a bottom-base 28 which is adjacent the cover 27 of the container unit when both units are connected (see Fig. 3). The functional unit B comprises mechanical mechanisms for generating the negative pressure and electrical means for measuring and/or indicating the vacuum level inside the container unit. For instance, a battery and/or a rechargeable battery 8 and/or a solar cell and/or a capacitor and optionally a pressure-level and charge-level display 10 may be integrated therein. An electronic circuit provided on a circuit board 23 controls an electric pressure gauge sensor and/or alarm indicating means and/or the display 10 for indicating the measured vacuum level. Corresponding with the said display 10, preferably LED lamps serving optical alarms preferably indicating loss of negative pressure in the container and low level in any of the said current accumulators (power sources). A means serving acoustical alarm is preferably attached to the circuit board 23. The functional unit with the electronic components, in particular the circuit board. 23, is preferably easily dismountable such that the individual components of the functional unit may be discharged or recycled separately.

By using the hand lever 1 (or turning knob or crank), negative pressure can be produced in the container 14 by a suction bellows 4 (suction bulb, elastic hollow space formed as bubble) or a piston or a paddle wheel. This negative pressure (vacuum) is set preferably between -25 mmHg (∼ -0.033 bar) and -600 mmHg (∼ -0.80 bar) depending on the indication or mode of application.

As already discussed above, according to a preferred operating mode, at the same time a dynamo 7 may be powered which charges the rechargeable battery 8 or a capacitor. The rechargeable battery or capacitor is delivering the power for the pressure display 10 and the optical alarm- or warning-LEDs 10 and/or acoustical error message when the negative pressure was fallen under a certain level. The power source can also be a solar cell or a battery. However, the operation of the hand lever 1 or crank may also result in a sole operation of the dynamo or a sole operation of the mechanical pump or both.

In order to abruptly let air into the container in case of emergency to abort or reduce the negative pressure, a (bleed-) valve 21, preferably made of red plastics, is affixed on the upper side of the functional unit. This valve can also be operated in order to reduce the negative pressure or suction in the container when it was inadvertently pumped beyond the aimed level of suction.

According to a preferred embodiment of the present invention, the functional unit B and the container unit A may be sold separately. Before use, the functional unit B has to be attached to the container A and hermetically sealed. This can be effected by o-ring seal and cramps or spring locks or screw top or by bayonet cap. A preferred embodiment of the present invention is a container A that is airtight and germ proof without being attached to the functional unit 13 comprising a container cover glued in place. If required the connection can be released again, for example when the container 14 is full and has to be changed. The preferably sterilely packed functional unit is designed in a way that it can be reused after corresponding cleaning and disinfection.

Two eyes (not shown in the drawings) may be affixed to the sides of the functional unit B to allow the carrying of the vacuum means by a shoulder strap.

The mode of operation will be described with reference to a preferred embodiment of the present invention as illustrated in the drawings.

After the pivot-mounted hand lever 1 is released with the catch 2 for the hand lever, the hand lever 1 is automatically pushed out of the housing of the functional unit A through the twisted leg spring 3. Thereby, the suction bellows 4 is compressed. The air contained in the suction bellows 4 leaks via the outlet valve through the housing into the outside.

As long as the pin 5 is not pushed (see e.g. Fig. 4), only the flywheel 6 is rotated by a gear rack on the hand lever when using the hand lever 1. This flywheel 6 runs the dynamo 7 via an actuator. The dynamo charges the rechargeable batteries 8. The suction bellows 4 is thus opened but the air is not sucked out of the container 14 (closed fluid-collection container) but via the non-hermetic housing 18 of the functional unit A out of the room. Thus, no vacuum is produced. By means of the rechargeable batteries a pressure sensor 20, a preferably LCD display 10 for strength of the vacuum and charge level of battery as well as two warning-/alaT-m-LEDs are operated.

When the pin 5 is pushed (see Fig. 4), air is sucked out of the container 14 due to the opening of the suction bellows and thus negative pressure is produced. In particular, as shown in Figure 4, a pushed pin 5 enables a fluid (gas and/or liquid) communication from the inside of the container 14 via notch 17a to the filtration chamber 16, via the filter 9 to the pre-chamber 15 and further via groove or bore 12 to the suction bellows 4. When the pin 5 is not pushed it is biased by spring(s) 11a and 11b in a close position, i.e., the filtration chamber 16 is sealed or closed against the container via pin 17 and o-ring 30. As illustrated in Fig. 4, the pin 5, mounted at the functional unit, and the pin 17, mounted at the container unit, are coaxially aligned. Thus, when pin 5 is pushed down also pin 17 is pushed down. The sealable filtration chamber 16 with filter 9 provides an automatic protection of the hydrophobic bacteria filter. Thus, it is possible to carry out the therapy in a safe way and without restrictions or pauses even when the vacuum source is strongly moved, tilted, turned over or lying in the patient's bed someway.

The advantageous complete independency of a power network (mains) is achieved by the application of mechanically operated hand lever 1 and dynamo 7. The pin 5 allows a simultaneous or separated production of power for charging the rechargeable battery and of vacuum. The production of vacuum is also possible if any or all electrical components happens to be faulty - wound suction can be maintained.

Figure 4 shows the details of the valve in a cross sectional view. The valve comprises two coaxially arranged pins, namely a pin 5 in the functional unit B, and a pin 17 in the container unit A, wherein pin 17 is directly operated when pin 5 is pushed.

In the functional unit B there is a pin 5 with leading nose 5a which is pushed against the lower side of the lid via compression spring 11. The leading nose protects the pin 5 from twisting. The groove 12 contained in the pin 5 is in the original state (when the pin is not pushed) arranged in a way that the air inlet 13 of the suction bellows 4 is opened outwardly. That means that in this state an opening of the bellows does not result in the production of negative pressure in the container 14.

When the pin 5 is pushed, the groove 12 with borehole slides towards the opening of the pre-chamber 15. The necessary connection to the suction bellows 4 is thereby generated. The pre-chamber is hermetically connected to the filtration chamber 16 of the container A via an o-ring 19. In the filtration chamber there is another pin 17 which is slid at the same time so that access to the container 14 is generated via a corresponding groove. Thus, by opening of the suction bellows, air can be sucked out of the container in order to establish the necessary negative pressure. Due to a hydrophobic bacteria filter 9 on the upper side of the filtration chamber 16 the air is germ-free before entering the suction bellows. In this embodiment, the pin must be pushed and held in order to activate the hydrophobic bacteria filter 9 in the suction circuit and to produce a vacuum in the container. When the pin is not pushed or released, the bacteria filter is automatically protected and out of order.

According to a further preferred embodiment, it is also possible to arrest the pin in the pushed state, e.g., by twisting the pin 5 such that a permanent pushing during suction is not necessary. When the desired level of vacuum is achieved, the arrest is released, e.g., by twisting the pin in the other direction (primary setting).

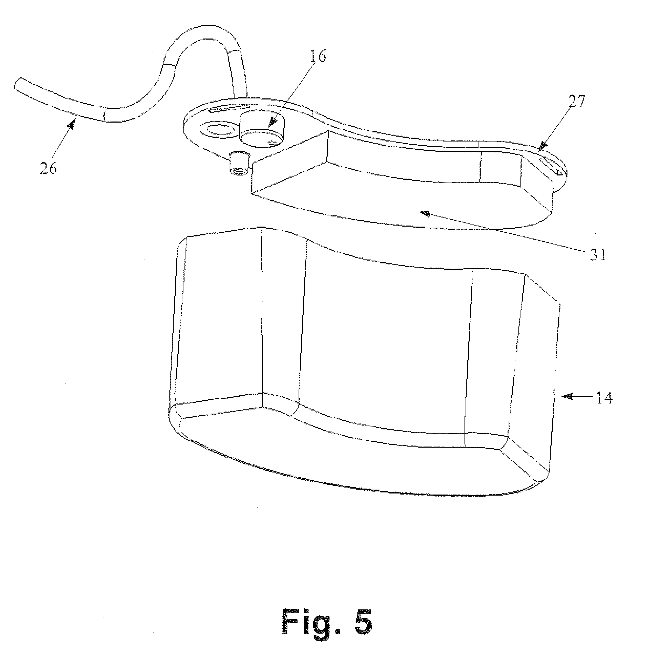

Figure 5 shows an exploded view (from below) of a container unit A. The container unit according to this particular embodiment comprises a container 14 and a cover 27 for seating the container, preferably germ-proof sealing. A suction hose 26 for suction of wound exudate is mounted to the cover 27. A filter chamber 16 is located at the lower side of the cover 27. Moreover, an automatic vacuum adjustment or compensation means 31 is located at the lower side of the cover 27. The automatic vacuum adjustment means 31 comprises a changeable volume, which changes in response to the pressure applied to said vacuum adjustment means 31.

For instance, it is assumed that the volume of the container unit without a vacuum adjustment means 31 is V_{C} (intrinsic volume of the container unit). At normal atmospheric pressure, e.g., in case there is no vacuum inside the container, the volume of the vacuum adjustment means 31 is V₁. Thus, in case a vacuum adjustment means 31 is provided inside the container unit A, the (active) volume of the container unit A is reduced: Vc - V₁. When the pressure inside the container unit is reduced (negative pressure), the volume of the vacuum adjustment means changes, in particular, the volume of the vacuum adjustment means 31 preferably increases (V2>V1).

In negative pressure wound therapy, the vacuum level is adjusted in the beginning to the desired level. However, the wound exudate which flows inside the container changes the active volume inside the container unit A, i.e., the wound exudate reduces the active volume inside the container unit. Accordingly, the vacuum level changes, in particular, the vacuum level is reduced. When the vacuum level inside the container unit is reduced (the absolute pressure is increased), the volume of the vacuum adjustment means changes, in particular, the volume decreases such that the active volume inside the container unit increases. Accordingly, the change of the vacuum level inside the container unit is reduced or eliminated by using the vacuum adjustment means.

The automatic vacuum adjustment means 31 comprises preferably cellular foam, for instance closed cell structure foam. In other words, it is preferred that the foam comprises closed cells (bubbles) with gas inside. A plurality of different kinds of closed cell foam may be used. However, it is preferred that the foam material is soft such that the compression load deflection (also called: indentation hardness or indentation force deflection (IFD); in German: Stauchhärte; see e.g., DIN 53577 or ISO 3386 ) is low, preferably <100kPa, more preferably <50kPa and more preferably <30kPa or even less. Without being limited to a particular material, suitable materials are for example: synthetic rubber (elastomer), cellular caoutchouc, EPDM (Ethylene-Propylene-Dien-caoutchouc) and/or CR (Chloroprene-caoutchouc; "NEOPREN®").

The invention has been illustrated and described in detail in the drawings and foregoing description. Such illustration and description are to be considered in an illustrative or exemplary and non-restrictive manner, i.e., the invention is not limited to the disclosed embodiments. Moreover, the word "comprising" does not exclude other elements or steps, and the indefinite article or "an" does not exclude a plurality. A single processor or other unit may fulfil the functions of several items recited in the claims. The mere fact that certain measures are recited in mutually different dependent claims does not indicate that a combination of these measures cannot be used to advantage. Any reference signs in the claims should not be considered as limiting the scope.

## Claims

1. A fluid evacuator for negative pressure wound therapy comprising:
a container unit (A) and a functional unit (B);
wherein said container unit (A) comprises at least a container (14) for the collection of wound fluids; and
said functional unit (B) is adapted for controlling the pressure inside said container (14), said functional unit (B) comprising a manual pump for creating a vacuum inside the container (14),
wherein the functional unit (B) further comprises an electric pressure gauge for indicating the pressure inside the container (14).

2. The fluid evacuator according to claim 1, wherein the container unit (A) and the functional unit (B) are detachably or firmly connected.

3. The fluid evacuator according to claim 1 or 2, wherein the manual pump comprises a suction bellows (4), a piston and/or a paddle wheel for evacuating the container (14).

4. The fluid evacuator according to claim 3, wherein the functional unit (B) comprises a housing (18) and the manual pump is operable by means of a lever (1), wherein said lever (1) is preferably pivotally supported and preferably concealable within said housing (18).

5. The fluid evacuator according to claim 4, wherein the lever is biased to extend beyond the housing, wherein the suction bellows (4) is preferably compressed when the lever extends beyond the housing.

6. The fluid evacuator according to any of claims 4 or 5, wherein the suction bellows (4) expands when the lever (1) moves toward the housing, such that air is evacuated from the container (14).

7. The fluid evacuator according to any of the preceding claims, wherein the functional unit (13) further comprises a battery, a rechargeable battery (8) and/or capacitor and/or a solar cell and/or a dynamo (7) for providing electrical power.

8. The fluid evacuator according to claim 7, wherein the operation of the lever (1) drives the dynamo (7) for generating electrical power, wherein said electrical power is preferably used to charge the rechargeable battery (8) and/or a capacitor.

9. The fluid evacuator according to any of claims 4 to 8, wherein the functional unit (B) and/or the container unit (A) comprises a mechanism which ensures that the container is not evacuated when the lever- (1) is operated.

10. The fluid evacuator according to any of the preceding claims, wherein the functional unit (B) comprises a mechanical and/or electrical alarm indicator, and the electric pressure gauge is preferably provided with a digital display (10) for displaying the pressure inside the container (14).

11. The fluid evacuator according to any of the preceding claims, wherein the container unit (A) is germ-proof and preferably comprises a filter (9), preferably a hydrophobic germ filter (9) for filtering the air which is evacuated from the container.

12. The fluid evacuator according to claim 11, wherein the filter (9) is located within a filtration chamber (16) and wherein said filtration chamber (16) is preferably sealable against the container by means of a mechanically operable valve.

13. The fluid evacuator according to any of the preceding claims, wherein the container unit (A) comprises an automatic vacuum compensation means (31) with a displacement volume, wherein said displacement volume automatically decreases when the pressure inside the container increases.

14. the fluid evacuator according to any of the preceding claims, wherein a sachet (24) with a gelling agent is provided inside the container (14) preferably attached flat at the bottom of the container (14).

15. A method for generating negative pressure, which may be used for negative pressure wound therapy and exudate management, the method comprising the steps:
providing a fluid evacuator according to any of claims 1 to 14;
operating the manual pump until the electric pressure gauge indicates the desired negative pressure inside the container unit (14).
